# EUROPEAN PATENT SPECIFICATION

(11) **EP 3 429 456 B1**
(45) Date of publication and mention of the grant of the patent: **10.05.2023**
(21) Application number: 17708829.1
(22) Date of filing: 09.03.2017
(51) Int. Cl.: A61B 5/00

(54) **A METHOD AND APPARATUS FOR DETERMINING A BASELINE FOR ONE OR MORE PHYSIOLOGICAL CHARACTERISTICS OF A SUBJECT**
VERFAHREN UND VORRICHTUNG ZUR BESTIMMUNG EINER MESSBASIS FÜR EINE ODER MEHRERE PHYSIOLOGISCHE EIGENSCHAFTEN EINER PERSON
PROCÉDÉ ET APPAREIL PERMETTANT DE DÉTERMINER UNE VALEUR DE RÉFÉRENCE POUR UNE OU PLUSIEURS CARACTÉRISTIQUES PHYSIOLOGIQUES D'UN SUJET

(30) Priority: 15.03.2016 EP 16160468
(43) Date of publication of application: 23.01.2019
(73) Proprietor: Koninklijke Philips N.V., 5656 AG Eindhoven (NL)
(72) Inventor: MUEHLSTEFF, Jens, 5656 AE Eindhoven (NL); WEDA, Johannes, 5656 AE Eindhoven (NL); ATALLAH, Louis Nicolas, 5656 AE Eindhoven (NL); VAN DER HEIDE, Esther Marjan, 5656 AE Eindhoven (NL)
(74) Representative: Philips Intellectual Property & Standards
(86) International application number: PCT/EP2017/055502
(87) International publication number: WO 2017/157746

(56) References cited:
- EP-A1- 2 095 798
- WO-A2-2009/063463
- CN-A- 105 264 585

## Description

### Technical Field of the Invention

The invention relates to the field of measuring one or more physiological characteristics of a subject and, in particular, determining a baseline for the one or more physiological characteristics of the subject.

### Background to the Invention

The quantification of pain and stress of a subject has been a clinical need for a long time. Recently, physiological characteristics (including vital-signs such as heart rate, blood pressure and the like) have been used to objectify a response of a subject or patient to painful stimuli for this purpose. The basic underlying mechanism is that pain stimuli are known to activate the autonomic nervous systems and have an impact on physiological characteristics. Therefore, a change in the pain or stress level of a subject can sometimes be determined by observing a change in the physiological characteristics of the subject.

An example of using the physiological characteristics of a subject in assessing the physical or emotional state of a patient includes using electrocardiography (ECG) and photo-plethysmography (PPG) to estimate a pulse transit time (PTT) from the heart to the hand of a subject and perceiving an increase in PTT as an indicator of stress and pain. Another example includes extracting features from a combination of PPG with galvanic skin response (GSR) and transforming those features into a single measure that is used to assess pain. Other examples include using frequency features of heart rate variability obtained from ECG or a cardiac rhythm variability, respiratory sinus arrhythmia changes due to pain, blood pressure (BP) changes, or the like as indicators that a subject is experiencing pain and/or stress.

However, the normal range of physiological characteristics can vary considerably between subjects due to external factors. For example, blood pressure and heart rate can vary due to age, medical condition, environmental conditions, and other external factors. It is therefore necessary to normalise and clean acquired data before it can be used to infer reliable details on the physical and emotional state of the subject (for example, whether the subject is in pain, distressed, anxious, and so on).

Normalisation procedures are often based on time periods in which a subject is in a well-defined emotional and/or physical state without transitions (i.e. a time period in which the subject is at rest without potentially painful or emotional stimuli). For example, WO 2009/063463 discloses recording a baseline for a patient in a constant position (similar to that of treatment) with no/minimal pain stimuli. However, scheduling in a dedicated period in which the subject is at rest requires extra effort from a healthcare professional and reduces the efficiency of subject processing. Other methods are based on medical records, population statistics that are later customised per subject, or arbitrary thresholds set by clinicians. However, relying on historical or statistical subject records or setting arbitrary thresholds can significantly reduce the reliability of results. EP2095798 discloses a method of analyzing data from an implantable restriction device to determine a baseline value for a physiological parameter, comprising: collecting data from an implantable restriction device over a time period, the collected data containing information about values of a parameter sensed within a body over the time period; defining a range of values to represent a tolerance range; and comparing one or more values of the sensed parameter during the time period to the tolerance range to determine if all of the one or more values fell within the tolerance range, and if so, identifying a baseline as having been established.

Other methods use a visual analogue scale to allow subjects themselves to indicate their pain level. However, this method is only useful for subjects that are awake (i.e. not those that are sedated) and the required attention of the subject towards feeling pain can bias the estimation, making the assessment unreliable. The reliability can be increased by implementing methods that do not require the attention of the subject. However, the problem of there being a large variability in physiological characteristics between different subjects still remains.

Therefore, there is a need for a method and apparatus that can automatically and reliably determine a baseline (i.e. a suitable, undisturbed period) for a subject for use in normalising physiological characteristics acquired from the subject, which overcomes the problems of subject variability (such as differences in physiologies and conditions between subjects).

### Summary of the Invention

The invention is defined in the appended claims.

As noted above, a limitation with physiological characteristic measurement is that physiological characteristics are subject dependent and can be influenced by a range of external factors. In order to overcome these problems, it would be valuable to have a more reliable baseline for use in normalising physiological characteristics acquired from a subject.

Therefore, according to a first aspect of the invention, there is provided a method for determining a baseline for one or more physiological characteristics of a subject, the method comprising: acquiring one or more physiological characteristics of a subject and contextual information associated with the subject; detecting a time period in which the subject is in a baseline state based on the acquired one or more physiological characteristics of the subject and the contextual information associated with the subject; and determining the baseline for the one or more physiological characteristics of the subject from a value of at least one of the acquired one or more physiological characteristics in the detected time period.

According to the invention, the time period in which the subject is in a baseline state is a time period in which the acquired one or more physiological characteristics of the subject and the contextual information associated with the subject are within a set range or meet a set condition.

In some embodiments, the method may further comprise normalising the acquired one or more physiological characteristics.

In some embodiments, the method may further comprise comparing the acquired one or more physiological characteristics with the determined baseline.

In some embodiments, the method may further comprise determining if the subject is experiencing a level of pain and/or stress based on the comparison of the acquired one or more physiological characteristics with the determined baseline.

In some embodiments, determining if the subject is experiencing a level of pain and/or stress may comprise determining that the subject is experiencing a level of pain and/or stress if the variation of the acquired one or more physiological characteristics from the determined baseline is outside the set range.

In some embodiments, determining if the subject is experiencing a level of pain and/or stress may comprise determining that the subject is not experiencing a level of pain and/or stress if the variation of the acquired one or more physiological characteristics from the determined baseline is within the set range.

In some embodiments, the one or more physiological characteristics of the subject may comprise at least one of: a blood pressure measurement of the subject, a skin conductivity measurement of the subject, a heart rate measurement of the subject, a brain activity measurement of the subject, a breathing rate measurement of the subject, a muscle activity measurement of the subject, and a skin temperature measurement of the subject.

In some embodiments, the contextual information associated with the subject may comprise non-physiological related information.

In some embodiments, the contextual information associated with the subject may comprise information associated with at least one of: a posture of the subject, a movement of the subject, an emotional state of the subject, an environment condition for the subject, and a medication condition for the subject.

According to a second aspect of the invention, there is provided a computer program product comprising a computer readable medium, the computer readable medium having computer readable code embodied therein, the computer readable code being configured such that, on execution by a suitable computer or processor, the computer or processor is caused to perform the method of the methods described above.

According to a third aspect of the invention, there is provided an apparatus for determining a baseline for one or more physiological characteristics of a subject, the apparatus comprising a control unit configured to: acquire one or more physiological characteristics of a subject and contextual information associated with the subject; detect a time period in which the subject is in a baseline state based on the acquired one or more physiological characteristics of the subject and the contextual information associated with the subject; and determine the baseline for the one or more physiological characteristics of the subject from a value of at least one of the acquired one or more physiological characteristics in the detected time period.

In some embodiments, the control unit may be configured to acquire the one or more physiological characteristics of a subject and/or the contextual information associated with the subject from a memory unit in the apparatus.

In some embodiments, the control unit may be configured to acquire the one or more physiological characteristics of a subject by controlling a physiological characteristic sensor to measure the one or more physiological characteristics of the subject, and to acquire the contextual information associated with the subject by controlling a contextual information device to acquire the contextual information.

In some embodiments, the physiological characteristic sensor may be one or more of: a blood pressure sensor, a skin conductivity sensor, a heart rate sensor, a brain activity sensor, a breathing rate sensor, a muscle activity sensor, and a skin temperature sensor, and the contextual information device may be one or more of: a posture detection device for detecting a posture of the subject, a movement detection device for detecting movement of the subject, a user interface device for receiving a user input of an emotional state of the subject, an environment monitoring device for determining an environment condition for the subject, and a medication monitoring device for determining a medication condition for the subject.

It is thus possible to automatically and reliably determine a baseline for a subject for use in normalising physiological characteristics acquired from the subject, which overcomes the problems of subject variability. In understanding the baseline of a subject and comparing variations in acquired physiological characteristics of the subject to the baseline, a reliable indication of the physical and/or emotional state of the subject can be provided.

### Brief Description of the Drawings

For a better understanding of the invention, and to show more clearly how it may be carried into effect, reference will now be made, by way of example only, to the accompanying drawings, in which:
Figure 1 is a block diagram of an apparatus according to an embodiment of the invention;
Figure 2 is a flow chart illustrating a method according to an embodiment of the invention;
Figure 3 is a graphical illustration of the physiological characteristic of a pulse rate of a subject and contextual information associated with the subject of an acceleration signal associated with the subject and the activity of the subject derived from the acceleration signal over time;
Figure 4 is a graphical illustration of the physiological characteristic of a heart rate of a subject "A" over time and a subject "B" over time;
Figure 5 is a graphical illustration of physiological characteristics of a subject including a body temperature of the subject and a photoplethysmography (PPG) amplitude of the subject and contextual information associated with the subject including an acceleration signal associated with the subject over time; and
Figure 6 is a graphical illustration of physiological characteristics of a subject including a heart rate (HR) of the subject and a mean blood pressure (MBP) of the subject and contextual information associated with the subject including a medication dosage administered to the subject over time.

### Detailed Description of the Preferred Embodiments

As noted above, the invention provides an automatic and reliable determination of a baseline for a subject for use in normalising physiological characteristics acquired from the subject, which overcomes the existing problems associated with the variable nature of physiological characteristics due to external factors.

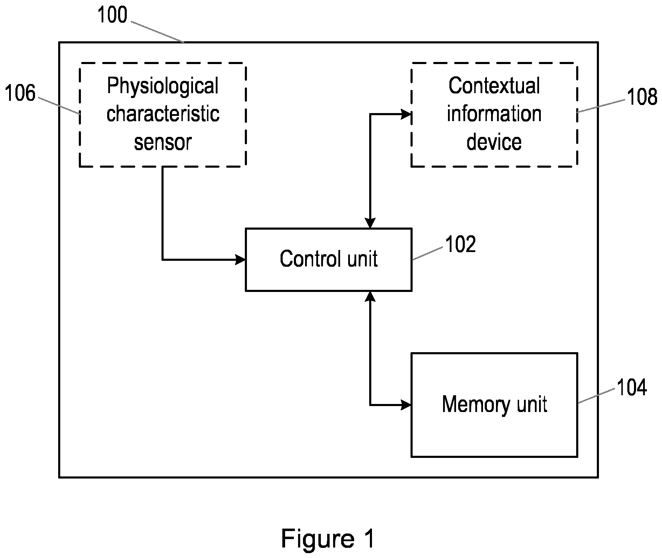

**Figure 1** shows a block diagram of an apparatus 100 according to an embodiment of the invention that can be used for determining a baseline for one or more physiological characteristics of a subject.

The apparatus 100 comprises a control unit 102 that controls the operation of the apparatus 100 and that can implement the method describe herein. Briefly, the control unit 102 is configured to determine a baseline for one or more physiological characteristics for a subject from a value of at least one physiological characteristic in a detected time period in which the subject is in a baseline state (for example, a stable or steady state). The control unit 102 detects the time period in which the subject is in a baseline state based on one or more acquired physiological characteristics of the subject and acquired contextual information associated with the subject. In other words, the control unit 102 is configured to use one or more acquired physiological characteristics of a subject and acquired contextual information associated with the subject to detect a time period in which the subject is in a baseline state and determine a baseline for the one or more physiological characteristics for the subject from a value of at least one physiological characteristic in the detected time period.

A physiological characteristic of the subject is a measurable characteristic of a subject (such as a vital-sign) that is indicative of the health or state of the subject. A physiological characteristic may be affected by a level of pain (including discomfort) that the subject is experiencing, and/or a level of stress (including anxiety and/or alertness) that the subject is suffering. The contextual information associated with the subject comprises non-physiological related information (i.e. information different to the physiological characteristics). The contextual information is information that can put any detected change in acquired physiological characteristics into context. In other words, the contextual information is intended to provide intelligence on the physiological characteristics. For example, the contextual information is information that can be used to determine a cause or contributing factor of any change observed in the acquired physiological characteristics to determine whether the acquired physiological characteristics provide a reliable indication of the health/state of the subject, as explained in more detail later.

The control unit 102 can comprise one or more processors, processing units, multi-core processors or modules that are configured or programmed to control the apparatus 100 to determine a baseline for a physiological characteristic for the subject. In particular implementations, the control unit 102 can comprise a plurality of software and/or hardware modules that are each configured to perform, or are for performing, individual or multiple steps of the method according to embodiments of the invention.

The apparatus 100 comprises a memory unit 104 that can be used for storing program code that can be executed by the control unit 102 to perform the method described herein. The memory unit 104 can also be used to store information, signals and measurements made or acquired by any sensors or devices (including a physiological characteristic sensor 106 and/or a contextual information device 108) that are part of the apparatus 100 or that are external to the apparatus 100. In some embodiments, the control unit 102 may be configured to acquire the one or more physiological characteristics of a subject and/or contextual information associated with the subject from the memory unit 104 in the apparatus 100 or an external memory unit.

In some embodiments, the apparatus 100 comprises a physiological characteristic sensor 106 for acquiring one or more physiological characteristics of the subject and the control unit 102 may be configured to acquire the one or more physiological characteristics of a subject by controlling the physiological characteristic sensor 106 in the apparatus 100 to measure the at least one physiological characteristic of the subject. In other embodiments, the control unit 102 may be configured to acquire the one or more physiological characteristics of a subject from an external physiological characteristic sensor (i.e. a physiological characteristic sensor that is separate from the apparatus 100). In some embodiments, multiple physiological characteristics can be acquired by one or multiple physiological characteristic sensors.

In some embodiments, the physiological characteristic sensor 106 may be a blood pressure sensor for acquiring blood pressure measurements of the subject. The blood pressure sensor can be any type of device that is able to measure the blood pressure of a subject. For example, the blood pressure sensor can be in the form of a cuff-based device. In this example, a cuff is placed around a limb (for example, an arm) or digit (for example, a finger) of the subject, a pump is used to inflate the cuff to a desired pressure, and a pressure sensor measures the air pressure inside the cuff. The pump may also be configured to deflate the cuff to a desired pressure and/or the device can comprise a valve that can be controlled to deflate the cuff. A processor or control unit in the device (or alternatively the control unit 102) can analyse the measurements from the air pressure sensor and control the pump and/or valve to inflate and/or deflate the cuff accordingly to obtain a measurement of the blood pressure of the subject. In an example, the blood pressure of the subject can be acquired by measuring sound distal from the cuff (known as the auscultatory method) or by measuring pressure pulsations in the cuff caused by volume pulsations of the arm and brachial artery and extracting features from the envelope of these pressure pulses (known as the oscillometric method). Those skilled in the art will be aware of cuff-based devices and other types of devices (e.g. that do not use a cuff) that can be used to measure blood pressure.

In some embodiments, the physiological characteristic sensor 106 may be a skin conductivity sensor for acquiring skin conductivity measurements (i.e. galvanic skin response). Skin conductance can be measured using a pair of electrodes that are placed in contact with the skin of the subject. An electrical current may be applied through one of the electrodes and the resistance of the skin measured, or the voltage between the electrodes can be measured. In an exemplary embodiment, the electrodes can be configured to be in contact with the skin on the palm of a hand of the subject.

In some embodiments, the physiological characteristic sensor 106 may be a heart rate sensor for acquiring heart rate measurements of the subject. The heart rate sensor may be any type of heart rate sensor. In one example, the heart rate sensor may be an electrocardiogram (ECG) sensor and the heart rate measurement of the subject may be acquired from an ECG signal (i.e. a signal representing the electrical activity of the subject's heart) measured by the ECG sensor. The ECG sensor may comprise at least two electrodes. In the example of an ECG sensor comprising two electrodes, the electrodes may be located either side of the subject's heart (for example, located on each arm). However, those skilled in the art will be aware of other arrangements for the electrodes on the body of the subject to acquire a heart rate measurement of the subject. In another example, the heart rate sensor may be a photo-plethysmography (PPG) sensor and the heart rate measurement of the subject may be acquired from a PPG signal measured by the PPG sensor. In other examples, the heart rate sensor can be an accelerometer located on the chest or back of the subject (with an acceleration measurement signal being processed to identify accelerations/movements due to the beating of the heart), a microphone located on the chest or back of the subject (with the sound measurement signal being processed to identify sounds that occur due to the beating of the heart) or a blood oxygenation (SpO2) sensor.

Other examples of physiological characteristic sensors 106 can include a brain activity sensor for acquiring brain activity measurements (such as electrodes for acquiring electroencephalography, EEG, measurements) of the subject, a breathing (i.e. respiration) rate sensor for acquiring breathing rate measurements of the subject, a muscle activity sensor for acquiring muscle activity measurements of the subject (such as an electromyograph for acquiring electromyography, EMG, measurements) and a skin temperature sensor for acquiring skin temperature measurements of the subject. Although some examples have been provided for the physiological characteristic sensor 106 and the physiological characteristic, those skilled in the art will be aware of other physiological characteristic sensors 106 and other types of physiological characteristics that can be used. In some embodiments, a combination of physiological characteristic sensors 106 can be used to acquire more than one type of physiological characteristic.

In some embodiments, the apparatus 100 comprises a contextual information device 108 for acquiring contextual information associated with the subject, and the control unit 102 may be configured to acquire the contextual information associated with the subject by controlling the contextual information device 108 in the apparatus 100 to acquire the contextual information. In other embodiments, the control unit 102 may be configured to acquire the contextual information associated with the subject from an external contextual information device (i.e. a contextual information device that is separate from the apparatus 100). In some embodiments, multiple types of contextual information can be acquired by one or multiple contextual information devices.

In some embodiments, the contextual information device 108 may be a posture detection device for detecting a posture of the subject. The posture detection device can comprise a camera. For example, the camera may be directed at the subject to acquire images or a video of the subject and the posture of the subject can be determined from the acquired images or video. Alternatively or in addition, the posture detection device can comprise a pressure detection device. For example, the pressure detection device may comprise a plurality of pressure sensors placed at different locations on the surface on which the subject rests (for example, the surface of a table, bed, chair, or the like). The plurality of pressure sensors may be embedded in the surface itself or in a sensor mat placed on top of the surface. The plurality of pressure sensors can sense pressure caused by different areas of the body of the subject on the surface, which can then be used to estimate the posture (or pose) of the subject.

In some embodiments, the contextual information device 108 may be a movement detection device for detecting a movement of the subject. The movement detection device can comprise a camera. For example, the camera may be directed at the subject to acquire images or a video of the subject and the movement of the subject can be determined from the acquired images or video. Alternatively or in addition, the movement detection device can comprise an accelerometer sensor. The accelerometer sensor may be placed in the bed of the subject or may be a wearable accelerometer sensor for placement on the body of the subject. For example, the movement detection device may comprise a removable strap for placement onto the body of the subject and the removable strap may comprise the accelerometer sensor. The removable strap may be placed around the subject (such as, around a trunk, leg, or arm of the subject). The movement detection device can comprise more than one accelerometer sensor, each accelerometer sensor for placement at a different location in the bed of the subject or at a different location on the body of the subject.

Other examples of contextual information devices 108 and the contextual information can include a user interface device (such as a touch screen, a keyboard, a keypad, or the like) for receiving a user input of an emotional state of the subject, an environment monitoring device for determining an environment condition for the subject (such as a thermometer for monitoring the temperature of the environment), and a medication monitoring device for determining a medication condition for the subject (such as a dosage monitoring device for monitoring the dosage of medication administered to the subject, which in one example may be connected to an infusion pump that is delivering the medication to the subject or in another example may be connected to electronic medical records that indicate medication information for the subject). Although some examples have been provided for the contextual information device 108 and the contextual information, those skilled in the art will be aware of other contextual information device 108 and other types of contextual information that can be used. In some embodiments, a combination of contextual information devices 108 can be used to acquire more than one type of contextual information.

In some embodiments, the control unit 102 may be configured to acquire the one or more physiological characteristics of a subject and the contextual information associated with the subject from any combination of a memory unit 104 in the apparatus 100, an external memory unit, a physiological characteristic sensor 106 in the apparatus 100, an external physiological characteristic sensor, a contextual information device 108 in the apparatus 100, and an external contextual information device such as those described above.

The acquired one or more physiological characteristics of a subject and the acquired contextual information associated with the subject can be processed generally as the they are acquired (for example, in real-time or near-real time), or they can be stored in the memory unit 104 and the control unit 102 can retrieve and process the previously-acquired physiological characteristics and contextual information from the memory unit 104 at a later time. The output of the physiological characteristic sensor 106 may be a time series of values for the physiological characteristic, or 'raw' measurements (for example, measurements of heart rate, an ECG signal, and so on) that are processed by the control unit 102 in order to determine a time series of values for the physiological characteristic. Similarly, the output of the contextual information device 108 may be a time series of values for the contextual information, or 'raw' data (for example, environment temperature, medication dosage, and so on) that is processed by the control unit 102 in order to determine a time series of values for the contextual information.

In some embodiments, the control unit 102 (and thus the apparatus 100) may be part of a mobile device (such as a smart phone) or other general purpose computing device that can comprise, be connected to or otherwise acquire a signal or measurement from a physiological characteristic sensor 106 and a contextual information device 108. In these embodiments, depending on the physiological characteristic and contextual information to be acquired, the physiological characteristic sensor 106 and/or the contextual information device 108 may be integrated into the device or separate to the device but able to provide signals/measurements to the device for processing and analysis (for example, via a wired or wireless connection). In other embodiments, the apparatus 100 can be an apparatus that is dedicated to the purpose of acquiring physiological characteristics and contextual information.

It will be appreciated that Figure 1 only shows the components required to illustrate this aspect of the invention, and in a practical implementation the apparatus 100 may comprise additional components to those shown. For example, the apparatus 100 may comprise a battery or other power supply for powering the apparatus 100 or means for connecting the apparatus 100 to a mains power supply, and/or a communication module for enabling acquired physiological characteristics, contextual information, and/or determined baselines to be communicated to a base unit for the apparatus 100 or to a remote computer (for example, that is operated by a healthcare professional).

The apparatus 100 may also comprise at least one user interface component that is for use in providing the subject or other user of the apparatus 100 (for example, healthcare provider) with information resulting from the method according to the invention. The user interface component can comprise any component that is suitable for providing the information resulting from the method according to the invention, and can be, for example, any one or more of a display screen or other visual indicator, a speaker, one or more lights, and a component for providing tactile feedback (e.g. a vibration function). The user interface component may be or may comprise means that enables the subject or another user of the apparatus 100 to interact with and/or control the apparatus 100. For example, the user interface component could comprise a switch, a button or other control means for activating and deactivating the apparatus 100 and/or acquisition process.

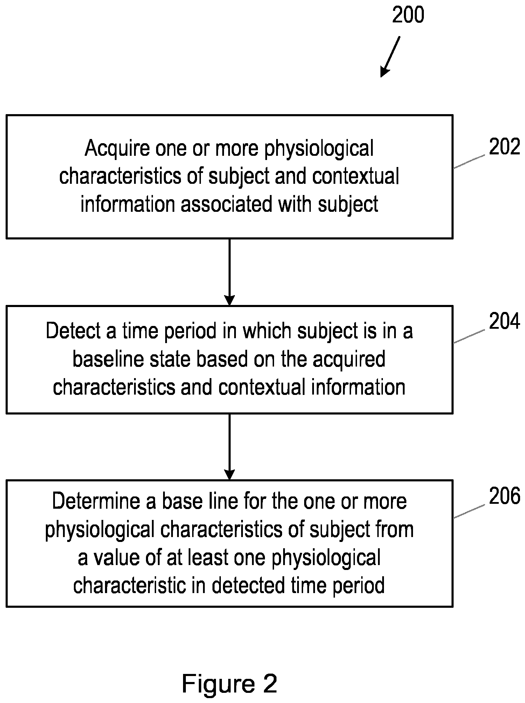

**Figure 2** illustrates a method 200 for determining a baseline for one or more physiological characteristics of a subject according to an embodiment of the invention. This method can generally be performed by or under the control of the control unit 102 of the apparatus 100.

With reference to Figure 2, at block 202, one or more physiological characteristics of a subject and contextual information associated with the subject is acquired. The one or more physiological characteristics of the subject can be acquired directly using a physiological characteristic sensor 106 (which can either be part of the apparatus 100 or separate to the apparatus 100, as described earlier) or the one or more physiological characteristics of the subject can be retrieved from the memory unit 104. Similarly, the contextual information associated with the subject can be acquired directly using a contextual information sensor 108 (which can either be part of the apparatus 100 or separate to the apparatus 100, as described earlier) or the contextual information associated with the subject can be retrieved from the memory unit 104. As described earlier, the outputs of the physiological characteristic sensor 106 and the contextual information device 108 may be a time series of values for the physiological characteristic and the contextual information, or 'raw' measurements that are processed by the control unit 102 in order to determine a time series of values.

At block 204, a time period in which the subject is in a baseline state is detected based on the acquired one or more physiological characteristics of the subject and the acquired contextual information associated with the subject. In other words, a baseline period is detected. The baseline period may be the time period in which the subject is determined, from analysis of the acquired one or more physiological characteristics of the subject and the acquired contextual information associated with the subject, to be experiencing a low level of stress and/or pain (or even no stress and/or pain).

As mentioned earlier, a physiological characteristic of the subject can be influenced by external factors such that acquired physiological characteristics may not provide a true or accurate indication of the health or state of the subject (such as the pain and/or stress level experience by the subject). For example, the external factors that may affect the accuracy of any acquired physiological characteristics may include a change in the environmental conditions to which the subject is exposed, a change in the medication concentration (such as pain relief medication, anaesthetics, or similar) in the subject, a change in the orientation or posture of the subject, a movement of the subject, or any other external factor. In order to improve the reliability of determining a baseline period for the subject, the determination takes into account contextual information in addition to physiological characteristics in order to determine any underlying cause or contributing factor to a change observed in the physiological characteristics of a subject and reliably determine a baseline period that reflects the true health/state of the subject.

The time period in which the subject is in a baseline state may be determined as a time period in which the acquired one or more physiological characteristics of the subject and the contextual information associated with the subject is within a set range or meets a set condition. For example, the time period in which the subject is in a baseline state may be a time period in which the acquired one or more physiological characteristics of the subject is above a minimum threshold value, below a maximum threshold value, constant (i.e. a variation in the blood pressure of the subject is below a threshold value), meets a condition set by an operator such as a professional healthcare provider or any combination thereof. In addition, the time period in which the subject is in a baseline state may be a time period in which the acquired contextual information associated with the subject is above a minimum threshold value, below a maximum threshold value, constant (i.e. a variation in the blood pressure of the subject is below a threshold value), meets a condition set by an operator such as a professional healthcare provider, or any combination thereof. In this way, it is possible to detect a time period in which the subject is in a baseline state where the acquired one or more physiological characteristics are in a stable state and there is no (or minimal) influence on the acquired physiological characteristics from external factors.

Some examples for determining that the acquired one or more physiological characteristics of the subject are within a set range or meet a set condition include determining any one or more (and any combination) of the following:
- a blood pressure measurement of the subject is above a minimum threshold value, below a maximum threshold value and/or is constant (i.e. a variation in the blood pressure of the subject is below a threshold value);
- a skin conductance measurement (i.e. the galvanic skin response) of the subject is below a threshold value and/or is constant (i.e. a variation in the skin conductance of the subject is below a threshold value);
- a heart rate measurement of the subject is above a minimum threshold value, below a maximum threshold value and/or is constant (i.e. a variation in the heart rate of the subject is below a threshold value);
- a brain activity measurement of the subject is above a minimum threshold value, below a maximum threshold value and/or is constant (i.e. a variation in the brain activity of the subject is below a threshold value);
- a breathing rate measurement of the subject is above a minimum threshold value, below a maximum threshold value and/or is constant (i.e. a variation in the breathing rate of the subject is below a threshold value);
- a muscle activity measurement of the subject is above a minimum threshold value, below a maximum threshold value and/or is constant (i.e. a variation in the muscle activity of the subject is below a threshold value); and
- a skin temperature measurement of the subject is above a minimum threshold value, below a maximum threshold value and/or is constant (i.e. a variation in the skin temperature of the subject is below a threshold value).

In one embodiment, the threshold values mentioned above for the physiological characteristics of the subject can be obtained by a population-based analysis of subjects with similar characteristics. In this embodiment, the median (or mean) of the population can be used as an average value and upper and lower limits can be statistically selected for use as the maximum and minimum threshold values respectively. For example, if an acquired physiological characteristic has a value that falls within a distance of +/- twice the standard deviation, the acquired physiological characteristic is determined to be within a set range or to meet a set condition (i.e. the acquired physiological characteristic is determined to be "normal").

In another embodiment, previously acquired data of the same subject can be used to set the threshold values. In another embodiment, the threshold values may be set based on values for one or more physiological characteristics of the subject acquired during an initial monitoring period of the subject (for example, a period in which the subject is at rest, performing physical activity, and/or under medication). The acquired values for the one or more physiological characteristics of the subject may be acquired from measurements performed during a preparation phase of the subject and/or may be acquired from an electronic medical record for the subject.

In addition, some examples for determining that the acquired contextual information associated with the subject is within a set range or meets a set condition include determining any one or more (and any combination) of the following:
- an orientation or posture of the subject is constant (i.e. a variation in the orientation or posture of the subject is below a threshold value);
- a movement of the subject is below a threshold value;
- an emotional state of the subject is positive (i.e. above a threshold value, which may be indicated on a scale or the like);
- an environment condition for the subject is met such as a temperature of the environment for the subject is above a minimum threshold value, below a maximum threshold value and/or is constant (i.e. a variation in the environment condition for the subject is below a threshold value), the number of healthcare professionals in the proximity of the subject is less than a threshold value, a sound level in the proximity of the patient is below a maximum threshold value, or similar; and
- a medication condition for the subject is met such as the medication dosage is above a threshold value, a time period from administering medication to the subject has expired, or similar.

In the example in which the acquired contextual information associated with the subject is determined to be within a set range (or meet a set condition) because the variation in the orientation or posture of the subject is below a threshold value, the threshold value for the subject may be derived from a period in which the subject is in a relaxed state (for example, a period in which the subject is lying down, sitting or in some other relaxed state). This period may be detected where there is a period of consistent values for the acquired physiological characteristics followed by a rise in the acquired values. In this example, the period of consistent acquired values is an indication of a relaxed period (such as a period in which the subject is lying/sitting down) whereas the rise in the acquired values is an indication of a disturbed period (such as a period where the subject moves from the lying/sitting down position). The period of consistent acquired values, before the rise in acquired values, is used to derive the threshold value for the subject in this example.

In the example in which the acquired contextual information associated with the subject is determined to be within a set range (or meet a set condition) because a movement of the subject is below a threshold value, the threshold value for the subject may be derived from a period of low activity. For example, there may be a continuous mode of acquiring activity measurements of the subject and the acquired activity measurements may then be classified into different levels of activity (such as low, medium and high). This can be done over time windows (for example, a time window of a few minutes). The acquired activity measurements classified into the lowest level of activity can then be used to derive the threshold value for the subject. For example, a low level of activity may be used to derive a minimum threshold value for the heart rate of the subject (which may be a value below 70 beats/min such as 67 beats/min, 65 beats/min, 60 beats/min, 50 beats/min, 40 beats/min, or similar) and a high level of activity may be used to derive a maximum threshold value for the heart rate of the subject (which may be a value above 140 beats/min such as 145 beats/min, 150 beats/min, 155 beats/min, or similar).

In the example in which the acquired contextual information associated with the subject is determined to be within a set range (or meet a set condition) because a medication condition for the subject is met, the medication may be any medication that may affect the physiological characteristics of the subject. For example, the medication may be a pain reduction medication (such as an opioid or the like), blood thinning medication, sedation medication, muscle relaxant medication, or any other medication that may affect the physiological characteristics of the subject. The threshold value for the medication dosage may be a standard value (for example, a value set by an expert) and may take into consideration subject characteristics (for example, age, weight, condition, or similar).

Although some examples are provided above for a determination of the acquired one or more physiological characteristics of the subject and the contextual information associated with the subject being within a set range or meeting a set condition, those skilled in the art will be aware of other possibilities.

The time period in which the subject is in a baseline state may be detected automatically (for example, in real-time or near real-time) on acquiring the one or more physiological characteristics of the subject and the acquired contextual information associated with the subject. Alternatively, where the acquired one or more physiological characteristics and the acquired contextual information associated with the subject is retrieved from the memory unit 104, the time period in which the subject is in a baseline state is detected at a different time to the acquisition.

At block 206, a baseline is determined for the one or more physiological characteristics for the subject from a value of at least one of the acquired one or more physiological characteristics in the detected time period. In other words, at least one of the physiological characteristics acquired during the detected time period is regarded as a baseline value for the acquired one or more physiological characteristics. In one example, where more than one value of the physiological characteristics acquired in the baseline period is used for determining the baseline, a mean or median value may be used as the baseline value for the acquired one or more physiological characteristics. In another example, the baseline value may be determined through voting or classification of the at least one physiological characteristic acquired during the detected time period. A determined baseline value is marked suitable for use in normalising the acquired one or more physiological characteristics and may be used as such.

For example, although not shown in Figure 2, after block 206, the acquired one or more physiological characteristics may then be normalised. Normalisation can be performed using any known normalisation technique. In one example, normalisation may involve averaging the acquired one or more physiological characteristics and then using the average to set upper and lower limits for the one or more physiological characteristics. The physiological characteristics falling within the upper and lower limits are the normalised physiological characteristics. In another example, normalisation may involve use of a mathematical expression (such as a logistic function, an arctan function, or similar) to rescale the one or more physiological characteristics to a fixed scale (such as a scale of 0 to 1). The rescaled one or more physiological characteristics are the normalised physiological characteristics. Although examples are provided for the normalisation technique that may be used to normalise the acquired one or more physiological characteristics of the subject, those skilled in the art will be aware of other normalisation technique.

In some embodiments, after block 206, the acquired one or more physiological characteristics (which can be the normalised physiological characteristics) may also be compared with the determined baseline. In one embodiment, it may be determined if the subject is experiencing a level of pain and/or stress based on the comparison of the acquired one or more physiological characteristics with the determined baseline.

If the variation of the acquired one or more physiological characteristics from the determined baseline is outside a set range, it is determined that the subject is experiencing a level of pain and/or stress. For example, if a measurement of a physiological characteristic exceeds the determined baseline by a certain amount (for example, by a certain percentage, such as 20%, 30%, 40%, 50% or any other percentage), then the measurement can be considered as indicating a high level of stress and/or pain.

On the other hand, if the variation of the acquired one or more physiological characteristics from the determined baseline is within a set range, it is determined that the subject is not experiencing a level of pain and/or stress or is experiencing a low level of pain and/or stress. For example, if a measurement of a physiological characteristic does not exceed the determined baseline by a certain amount (for example, by a certain percentage, such as 20%, 30%, 40%, 50% or any other percentage), then the measurement can be considered as indicating a that the subject is not experiencing a level of pain and/or stress or is experiencing a low level of pain and/or stress.

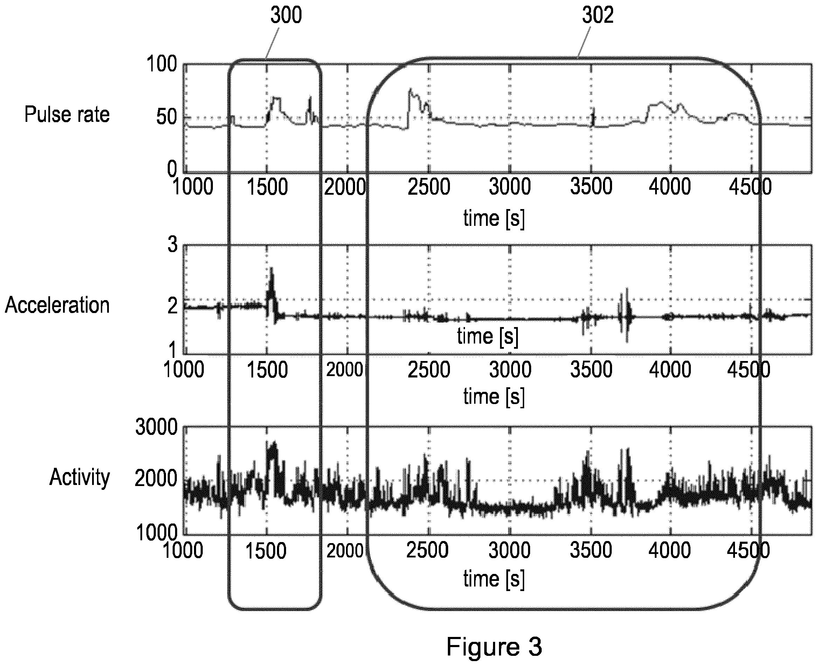

**Figure 3** is a graphical illustration of the physiological characteristic of a pulse rate of a subject (for example, acquired from a PPG) and contextual information associated with the subject of an acceleration signal associated with the subject (for example, acquired from an accelerometer) and the activity of the subject derived from the acceleration signal over time during preparation for a medical procedure.

The pulse rate of the subject during preparation for the medical procedure shows distinctive peaks at various times. From the accelerometer signal, it is apparent that the peaks in the pulse rate are related to the physical activity of the subject (i.e. the heart rate can be seen to increase due movement of the subject) and manipulations of the subject. A first set of peaks occurs in a first time period 300. In this example, the peaks in the first time period 300 are due to the subject climbing onto an operation table. A second set of peaks occur in a second time period 302. In this example, the peaks in the second time period 302 are due to manipulations on the subject (for example, injections, intubation, or the like). The manipulations are visible in the activity signal. There is no orientation change visible in the acceleration signal. It is thus determined that the pulse rate increase is due to pain and/or stress from manipulations on the subject. The first time period 300 and second time period 302 are determined to be unsuitable as baseline periods and the values of the pulse rate in these periods are determined to be unsuitable for use as a baseline for normalising the acquired pulse rate.

In another example, ambient sensors such as cameras or embedded sensors (such as pressure sensors) in the subject's bed can be used to indicate low activity periods. For example, it can be automatically detected from these sensors that the subject is lying in bed and either sleeping or doing a low activity. This period is taken as the baseline period and a value of a physiological characteristic in this baseline period is used as the baseline for normalisation.

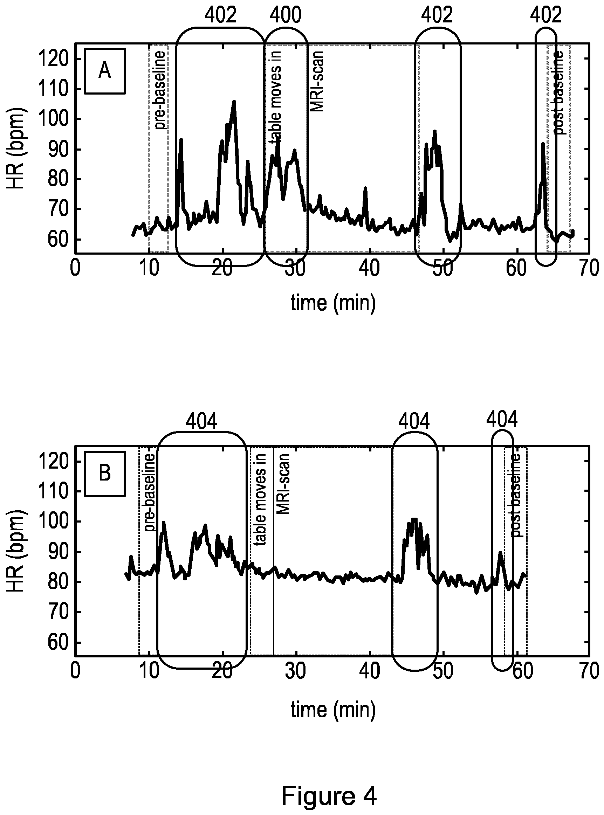

**Figure 4** is a graphical illustration of the physiological characteristic of a heart rate of a subject "A" over time and a subject "B" over time. In this example, the heart rate measurements are acquired during a magnetic resonance imaging (MRI) examination.

Several time periods of physical activity 402, 404 can be seen when the subjects move to/from the bed, to/from the MRI room, and onto/off the MRI table. When the table moves, the subject is required to lie perfectly still. It thus is determined that subject A was anxious (or stressed) in the time period before the examination 400 since a large increase in heart rate is observed during the time period in which subject A is lying still. On the other hand, it is determined that subject B was not anxious during this time period since no elevation in heart rate is observed in the time period in which subject B is lying still. It can also be determined that the heart rate of subject B is much higher than that of subject A during the baseline periods, thus emphasising the importance of normalisation. The contextual information acquired (relating to the movement of the subjects) prevents any confusion of heart rate increases related to physical activity, emotion, and pain. It is determined that the examination begins and ends with a baseline period in which the heart rate is low and stable without any influence from physical activity, emotion, and pain.

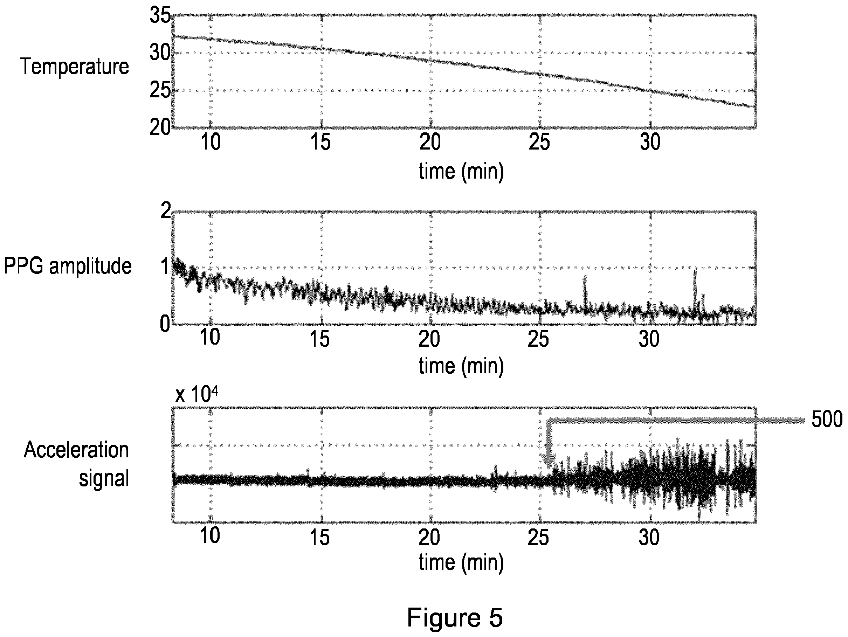

**Figure 5** is a graphical illustration of physiological characteristics of a subject including a body temperature of the subject and a photoplethysmography (PPG) amplitude of the subject and contextual information associated with the subject including an acceleration signal associated with the subject over time.

This example shows the impact of environmental temperature changes on amplitude of a PPG signal (acquired from the upper arm of the subject) and accelerometer signals (acquired via a wearable accelerometer on the trunk of the subject). The temperature measurements are acquired from ambient sensors and wearable sensors. The temperature can be seen to reduce over time. For example, this change may be due to the subject being moved to another room. The reduction in temperature causes vaso-motion, which has an impact on the PPG amplitudes. The accelerometer signals detect shivering of a subject at time 500 indicating that the subject regulatory system is responding to an environmental change and not pain or stress. Thus, from the increase in the amplitude of the acceleration at time 500, it can be determined that the PPG amplitude after said time 500 cannot be used as a baseline period. The combined analysis of contextual information (i.e. the acceleration information) and the physiological characteristics (i.e. the PPG and body temperature measurements) allow determination of a baseline period when a stable subject condition can be assumed for normalisation. The values of the PPG signal in the baseline period are used as a baseline for normalising the acquired pulse rate.

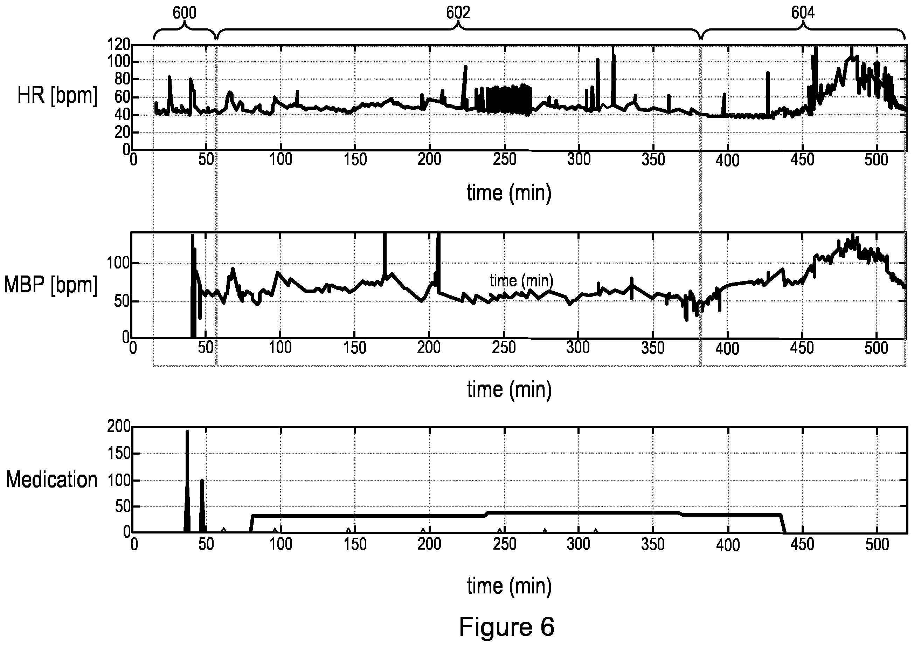

**Figure 6** is a graphical illustration of physiological characteristics of a subject including a heart rate (HR) of the subject and a mean blood pressure (MBP) of the subject and contextual information associated with the subject including a medication dosage administered to the subject over time before, during and after a medical procedure.

In this example, the dose of medication administered to the subject is controlled. For instance, the dosage of anaesthesia administered to the subject may be controlled by means of an anaesthesia machine. It is possible to automatically detect high concentration periods combined with the time the medication was administered to select a baseline period that can be used for normalisation of pain parameters. Where the concentrations of medication are high, the subject is sedated and this is combined with overall minimal values in acquired vitals (heart rate and mean blood pressure) for the subject. The peaks in the dosage of medication before the procedure indicate the start of surgery and, given that the medication has a known effect concentration (per weight), the required time is allowed to pass and then the period afterwards is detected as a low pain period that is used for normalisation.

There is therefore provided a method and apparatus for reliably determining a baseline for one or more physiological characteristics of a subject. The method and apparatus can be useful in monitoring a subject at home and in a professional healthcare facility (such as a hospital).

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. A computer implemented method (200) for determining a baseline for one or more physiological characteristics of a subject, the method comprising:
acquiring (202) from a physiological characteristic sensor or a memory unit one or more physiological characteristics of a subject and acquiring from a contextual information device or the memory unit contextual information associated with the subject;
detecting (204) a time period in which the subject is in a baseline state, namely a time period in which the acquired one or more physiological characteristics of the subject and the contextual information associated with the subject are within a set range or meet a set condition, wherein the contextual information associated with the subject comprises non-physiological related information that can put any detected change in the acquired one or more physiological characteristics of the subject into context; and
determining (206) the baseline for the one or more physiological characteristics of the subject from a value of at least one of the acquired one or more physiological characteristics in the detected time period.

2. A method (200) as claimed in claim 1, further comprising:
normalising the acquired one or more physiological characteristics.

3. A method (200) as claimed in claim 1 or 2, further comprising:
comparing the acquired one or more physiological characteristics with the determined baseline.

4. A method (200) as claimed in claim 3, further comprising:
determining if the subject is experiencing a level of pain and/or stress based on the comparison of the acquired one or more physiological characteristics with the determined baseline.

5. A method (200) as claimed in claim 4, wherein determining if the subject is experiencing a level of pain and/or stress comprises:
determining that the subject is experiencing a level of pain and/or stress if the variation of the acquired one or more physiological characteristics from the determined baseline is outside the set range.

6. A method (200) as claimed in claim 4, wherein determining if the subject is experiencing a level of pain and/or stress comprises:
determining that the subject is not experiencing a level of pain and/or stress if the variation of the acquired one or more physiological characteristics from the determined baseline is within the set range.

7. A method (200) as claimed in any preceding claim, wherein the one or more physiological characteristics of the subject comprises at least one of:
a blood pressure measurement of the subject;
a skin conductivity measurement of the subject;
a heart rate measurement of the subject;
a brain activity measurement of the subject;
a breathing rate measurement of the subject;
a muscle activity measurement of the subject; and
a skin temperature measurement of the subject.

8. A method (200) as claimed in any preceding claim, wherein the contextual information associated with the subject comprises non-physiological related information.

9. A method (200) as claimed in any preceding claim, wherein the contextual information associated with the subject comprises information associated with at least one of:
a posture of the subject;
a movement of the subject;
an emotional state of the subject;
an environment condition for the subject; and
a medication condition for the subject.

10. A computer program product comprising a computer readable medium, the computer readable medium having computer readable code embodied therein, the computer readable code being configured such that, on execution by a suitable computer or processor, the computer or processor is caused to perform the method of any of claims 1-9.

11. An apparatus (100) for determining a baseline for one or more physiological characteristics of a subject, the apparatus comprising:
a control unit (102) configured to:
acquire one or more physiological characteristics of a subject and contextual information associated with the subject;
detect a time period in which the subject is in a baseline state, namely a time period in which the acquired one or more physiological characteristics of the subject and the contextual information associated with the subject are within a set range or meet a set condition, wherein the contextual information associated with the subject comprises non-physiological related information that can put any detected change in the acquired one or more physiological characteristics of the subject into context; and
determine the baseline for the one or more physiological characteristics of the subject from a value of at least one of the acquired one or more physiological characteristics in the detected time period.

12. An apparatus (100) as claimed in claim 11, wherein the control unit (102) is configured to acquire the one or more physiological characteristics of a subject and/or the contextual information associated with the subject from a memory unit in the apparatus.

13. An apparatus (100) as claimed in claim 11, wherein the control unit (102) is configured to acquire the one or more physiological characteristics of a subject by controlling a physiological characteristic sensor to measure the one or more physiological characteristics of the subject, and to acquire the contextual information associated with the subject by controlling a contextual information device to acquire the contextual information.

14. An apparatus (100) as claimed in claim 13, wherein the physiological characteristic sensor is one or more of: a blood pressure sensor, a skin conductivity sensor, a heart rate sensor, a brain activity sensor, a breathing rate sensor, a muscle activity sensor, and a skin temperature sensor, and
wherein the contextual information device is one or more of: a posture detection device for detecting a posture of the subject, a movement detection device for detecting movement of the subject, a user interface device for receiving a user input of an emotional state of the subject, an environment monitoring device for determining an environment condition for the subject, and a medication monitoring device for determining a medication condition for the subject.

## Patentansprüche

1. Ein computerimplementiertes Verfahren (200) zur Bestimmung einer Basislinie für einen oder mehrere physiologische
Merkmale eines Patienten, wobei das Verfahren Folgendes umfasst:
Erfassen (202) 1 von einem Sensor für physiologische Merkmale oder einer Speichereinheit
eines oder mehrerer physiologischer Merkmale eines Patienten und Erfassen von Kontextinformationsgerät oder der Speichereinheit
für Kontextinformationen, die dem Patienten zugeordnet ist;
Erfassen (204) eines Zeitraums, in dem sich der Patient in einem Grundzustand befindet, d. h.
eine Zeitspanne, in der die erfassten ein oder mehreren physiologischen Merkmale des Patienten
und die mit dem Patienten verknüpften Kontextinformationen innerhalb eines festgelegten Bereichs liegen oder eine festgelegte Bedingung erfüllen, wobei die mit dem Patienten assoziierten Kontextinformationen nichtphysiologische bezogene Informationen umfassen, die jede erfasste Änderung der erfassten einen oder mehreren physiologischen Merkmale des Patienten in einen Kontext setzen können;
und
Bestimmen (206) der Basislinie für das eine oder die mehreren physiologischen
Merkmale des Patienten aus einem Wert von mindestens einem der erfassten ein oder mehreren
Merkmale des Patienten aus einem Wert von mindestens einem der erfassten ein oder mehreren.

2. Verfahren (200) nach Anspruch 1, der ferner Folgendes umfasst:
Normalisierung eines oder mehrerer erworbener physiologischer Merkmale.

3. Verfahren (200) nach Anspruch 1 oder 2, das ferner Folgendes umfasst:
Vergleich der erfassten ein oder mehreren physiologischen Merkmale mit der
ermittelten Basislinie.

4. Verfahren (200) nach Anspruch 3, das ferner Folgendes umfasst:
Bestimmung, ob der Patient einen Schmerz- und/oder Stresspegel erfährt, basierend auf
dem Vergleich des oder der erfassten physiologischen Merkmale mit der ermittelten
Basislinie.

5. Verfahren (200) nach Anspruch 4, wobei bestimmt wird, ob der Patient
Einem Schmerz- und/oder Stresspegel erfährt, das Folgendes umfasst:
Feststellung, dass der Patient einen Schmerz- und/oder Stresspegel erfährt, wenn die
Abweichung des oder der erfassten physiologischen Merkmale von der ermittelten
Basislinie außerhalb des festgelegten Bereichs liegt.

6. Verfahren (200) nach Anspruch 4, wobei bestimmt wird, ob der Patient Einen Schmerz- und/oder Stresspegel erfährt, das Folgendes umfasst:
Feststellung, dass die Person keinen Schmerz- und/oder Stresspegel erfährt, wenn
die Abweichung des erfassten einen oder mehrerer physiologischer Merkmale von der ermittelten Basislinie innerhalb des festgelegten Bereichs liegt.

7. Verfahren (200) nach einem der vorhergehenden Ansprüche, wobei der eine oder die mehreren physiologischen Eigenschaften des Patienten mindestens eines der folgenden Merkmale umfasst:
eine Blutdruckmessung bei dem Patienten;
eine Messung der Hautleitfähigkeit des Patienten;
eine Messung der Herzfrequenz des Patienten;
eine Messung der Gehirnaktivität des Patienten;
eine Messung der Atemfrequenz des Patienten;
eine Messung der Muskelaktivität des Patienten; und
eine Messung der Hauttemperatur des Patienten.

8. Verfahren (200) nach einem der vorhergehenden Ansprüche, wobei die Kontextinformationen,
die mit dem Patienten verbunden sind, nichtphysiologische Informationen umfassen.

9. Verfahren (200) nach einem der vorhergehenden Ansprüche, wobei die Kontextinformationen,
die mit dem Patienten, die mit dem Patienten verbunden sind, Informationen umfassen, die mit mindestens einem der folgenden Punkte verbunden sind:
eine Körperhaltung des Patienten;
eine Bewegung des Patienten;
einen emotionalen Zustand des Patienten;
eine Umgebungsbedingung für den Patienten; und
eine medikamentöse Behandlung des Patienten.

10. Ein Computerprogrammprodukt, das ein computerlesbares Medium umfasst, wobei das computerlesbares Medium einen computerlesbaren enthält, der so konfiguriert ist, dass er bei der Ausführung durch einen geeigneten Computer oder Prozessor,
der Computer oder Prozessor veranlasst wird, das Verfahren nach einem der Ansprüche 1-9 durchzuführen.

11. Ein Apparat (100) zur Bestimmung einer Basislinie für eine oder mehrere physiologische
Merkmale eines Patienten, wobei das Gerät Folgendes umfasst:
eine Steuereinheit (102), die dafür konfiguriert ist:
ein oder mehrere physiologische Merkmale eines Patienten und Kontextinformationen zu erfassen,
die mit dem Patienten verbunden sind;
einen Zeitraum erkennen, in der sich der Patient in einem Grundzustand befindet, d. h. einen
Zeitraum, in dem die erworbenen ein oder mehreren physiologischen Merkmale des Patienten und die Kontextinformationen, die mit dem Patienten verbunden sind, innerhalb eines festgelgten Bereichs liegen oder einer festgelegten Bedingung entsprechen,
wobei die mit dem Patienten assoziierten Kontextinformationen
nicht-physiologischen Informationen umfassen, die jede festgestellte Änderung in der erworbenen einen oder mehreren
physiologischen Merkmale des Patienten in einen Kontext stellen können;
und
Bestimmen der Basislinie für eine oder mehrere physiologische Eigenschaften des
Patienten aus einem Wert von mindestens einem der erfassten physiologischen Merkmale
in dem erfassten Zeitraum.

12. Apparat (100) nach Anspruch 11, wobei die Steuereinheit (102)
so konfiguriert ist, dass sie das eine oder die mehreren physiologischen Merkmale eines Patienten
und/oder die mit dem Patienten verbundenen Kontextinformationen aus einer Speichereinheit in dem Apparat erfasst.

13. Apparat (100) nach Anspruch 11, wobei die Steuereinheit (102)
dass sie das eine oder die mehreren physiologischen Merkmale eines Patienten erfasst, indem sie
einen Sensor für physiologische Merkmale steuert, um das eine oder die mehreren physiologischen Merkmale von dem Patienten zu messen
und zum Erfassen der mit dem Patienten verbundenen Kontextinformationen durch
Steuern eines Kontextinformationsgeräts zum Erfassen der Kontextinformationen.

14. Apparat (100) nach Anspruch 13, wobei die physiologische
Merkmale einer oder mehrere der folgenden Sensoren ist:
ein Blutdrucksensor, ein Hautleitfähigkeitssensor,
ein Herzfrequenzsensor, ein Gehirnaktivitätssensor, ein Atemfrequenzsensor, ein Muskelaktivitätssensor und
Ein Hauttemperatursensor und
wobei das Kontextinformationsgerät eines oder mehrere von Folgendem ist: ein Haltungserfassungsgerät zum Erfassen einer Körperhaltung
des Patienten, ein Bewegungserfassungsgerät zum Erfassen einer Bewegung des Patienten, ein
Benutzerschnittstellengerät zum Empfangen einer Benutzereingabe eines emotionalen Zustands des Patienten, ein Umgebungsüberwachungsgerät zum Bestimmen eines
Umgebungszustands für den Patienten und ein Medikationsüberwachungsgerät zum Bestimmen eines Medikationszustands für den Patienten.

## Revendications

1. Procédé mise en oeuvre par ordinateur (200) pour déterminer une ligne de base pour un ou plusieurs paramètres physiologiques
d'un sujet, le procédé comprenant:
l'acquisition (202) à partir d'un capteur de paramètres physiologiques ou d'une unité
de mémoire un ou plusieurs paramètres physiologiques d'un sujet et l'acquisition à partir d'une information contextuelle ou de l'unité de mémoire
des informations contextuelles associées au sujet;
détecter (204) une période de temps pendant laquelle le sujet est dans un état de base, à savoir
une période de temps pendant laquelle l'un ou plusieurs paramètres physiologiques acquis du sujet
et les informations contextuelles associées au sujet se situent dans une plage fixée ou répondent à une
condition fixée, dans laquelle les informations contextuelles associées au sujet comprennent des informations non
physiologiques qui peuvent mettre en relation tout changement détecté dans le ou les paramètres physiologiques acquis du sujet dans leur contexte;
et
déterminer (206) la ligne de base pour l'un ou plusieurs des paramètres physiologiques
du sujet à partir d'une valeur d'au moins l'un des un ou plusieurs
paramètres physiologiques acquis dans la période de temps détectée.

2. Procédé (200) selon la revendication 1, comprenant en outre:
la normalisation d'un ou plusieurs paramètres physiologiques acquis.

3. Procédé (200) selon la revendication 1 ou 2, comprenant en outre:
la comparaison d'un ou de plusieurs paramètres physiologiques acquis avec la ligne de base déterminée.

4. Procédé (200) selon la revendication 3, comprenant en outre:
déterminer si le sujet ressent un niveau de douleur et/ou de stress sur la base de
la comparaison d'un ou de plusieurs paramètres physiologiques acquis avec la ligne de base déterminée.

5. Procédé (200) selon la revendication 4, dans lequel déterminer si le sujet ressent un niveau de douleur et/ou de stress comprend:
déterminer que le sujet ressent un niveau de douleur et/ou de stress si la
variation d'un ou plusieurs paramètres physiologiques acquis par rapport à la ligne de base déterminée est en dehors de la plage fixée.

6. Procédé (200) selon la revendication 4, dans lequel déterminer si le sujet ressent
un niveau de douleur et/ou de stress comprend:
déterminer que le sujet ne ressent pas un niveau de douleur et/ou de stress si
la variation d'un ou de plusieurs paramètres physiologiques acquis par rapport à la ligne de base déterminée se situe dans la plage fixée.

7. Procédé (200) selon l'une quelconque des revendications précédentes, dans lequel le ou plusieurs paramètres physiologiques du sujet comprend au moins l'un des éléments suivants:
une mesure de la pression artérielle du sujet;
une mesure de la conductivité de la peau du sujet;
une mesure de la fréquence cardiaque du sujet;
une mesure de l'activité cérébrale du sujet;
une mesure de la fréquence respiratoire du sujet;
une mesure de l'activité musculaire du sujet; et
une mesure de la température de la peau du sujet.

8. Procédé (200) selon l'une quelconque des revendications précédentes, dans lequel les informations contextuelles
associées au sujet comprennent des informations non liées à la physiologie.

9. Procédé (200) selon l'une quelconque des revendications précédentes, dans lequel les informations contextuelles
associées au sujet comprennent des informations associées à au moins l'un des éléments suivants:
une posture du sujet;
un mouvement du sujet;
un état émotionnel du sujet;
une condition d'environnement pour le sujet; et
une condition de médication pour le sujet.

10. Produit de programme informatique comprenant un support lisible par ordinateur,
le support lisible par ordinateur comportant un code lisible par ordinateur incorporé dans celui-ci,
le code lisible par ordinateur étant configuré de telle sorte que, lors de l'exécution par un ordinateur ou un processeur
approprié, l'ordinateur ou le processeur est amené à exécuter le procédé de l'une quelconque des revendications 1 à 9.

11. Appareil (100) pour déterminer une ligne de base pour un ou plusieurs paramètres physiologiques d'un sujet, l'appareil comprenant:
une unité de commande (102) configurée pour:
acquérir un ou plusieurs paramètres physiologiques d'un sujet et des informations contextuelles associées au sujet;
détecter une période de temps pendant laquelle le sujet se trouve dans un état de base, à savoir une période de temps
au cours de laquelle le ou les paramètres physiologiques acquis du sujet et les informations contextuelles associées au sujet se situent dans une plage fixée ou répondent à une condition
fixée, dans laquelle les informations contextuelles associées au sujet comprennent des informations non physiologiques qui peuvent mettre en relation tout changement détecté dans le ou les paramètres physiologiques du sujet dans leur contexte; et
déterminer la ligne de base pour le ou les paramètres physiologiques du
sujet à partir d'une valeur d'au moins un ou plusieurs paramètres physiologiques
acquis dans la période de temps détectée.

12. Appareil (100) selon la revendication 11, dans lequel l'unité de commande (102) est
configurée pour acquérir un ou plusieurs paramètres physiologiques d'un sujet et/ou le
des informations contextuelles associées au sujet à partir d'une unité de mémoire dans l'appareil.

13. Appareil (100) selon la revendication 11, dans lequel l'unité de commande (102) est
configurée pour acquérir un ou plusieurs paramètres physiologiques d'un sujet en commandant un capteur de paramètres physiologiques pour mesurer un ou plusieurs paramètres physiologiques
du sujet, et pour acquérir les informations contextuelles associées au sujet en
commandant un dispositif d'informations contextuelles pour acquérir les informations contextuelles.

14. Appareil (100) selon la revendication 13, dans lequel le capteur de paramètres physiologiques
est un ou plusieurs des éléments suivants: un capteur de pression artérielle, un capteur
de conductivité de la peau, un capteur de fréquence cardiaque, un capteur d'activité cérébrale, un capteur de fréquence respiratoire, un capteur d'activité musculaire, et
un capteur de température de la peau, et
dans lequel le dispositif d'informations contextuelles est un ou plusieurs des éléments suivants: un dispositif
de détection de posture pour détecter une posture du sujet, un dispositif de détection de mouvement pour détecter un mouvement du sujet, un dispositif d'interface utilisateur destiné à recevoir une entrée utilisateur d'un état
émotionnel du sujet, un dispositif de surveillance de l'environnement pour déterminer une
condition d'environnement pour le sujet, et un dispositif de surveillance de la médication pour déterminer une
condition de médication pour le sujet.
